# EUROPÄISCHE PATENTSCHRIFT

(11) **EP 1 628 699 B1**
(45) Veröffentlichungstag und Bekanntmachung des Hinweises auf die Patenterteilung: **07.08.2013**
(21) Anmeldenummer: 04731323.4
(22) Anmeldetag: 06.05.2004
(51) Int. Cl.: A61M 16/04

(54) **TRACHEALBEATMUNGSVORRICHTUNG**
TRACHEAL VENTILATION DEVICE
DISPOSITIF DE RESPIRATION TRACHEALE

(30) Priorität: 15.05.2003 DE 10321990
(43) Veröffentlichungstag der Anmeldung: 01.03.2006
(73) Patentinhaber: KIMBERLY-CLARK WORLDWIDE, INC., Neenah, WI 54956 (US)
(72) Erfinder: GÖBEL, Fred, 69259 Wilhelmsfeld (DE)
(74) Vertreter: Leidescher, Thomas
(86) Internationale Anmeldenummer: PCT/EP2004/004797
(87) Internationale Veröffentlichungsnummer: WO 2004/101046

(56) Entgegenhaltungen:
- WO-A-01/34221
- WO-A1-01/02042
- DE-A- 19 845 415
- GB-A- 2 171 017
- US-A- 4 502 482
- US-A- 4 688 568
- US-B1- 6 287 290
- US-B1- 6 398 775

## Beschreibung

### Technisches Gebiet

Die Erfindung betrifft eine Trachealbeatmungsvorrichtung, insbesondere einen Trachealtubus, die die Trachea zum Beatmen eines pädiatrischen Patienten möglichst gewebeverträglich verschließt, mit einer die Trachea unterhalb der Glottis blockierenden Cuffblase, durch die eine Beatmungskanüle hindurchgeführt ist, wobei die Cuffblase aus einem flexiblem Weichfolienmaterial besteht und in gefülltem, ohne Begrenzung frei entfaltetem Zustand größer ist als in gefülltem Zustand in der Trachea plaziert und die Cuffblase mit Faltenwurf an der Trachea anliegt.

### Stand der Technik

In der DE 198 45 415 A1 ist eine Trachealbeatmungsvorrichtung beschrieben, bei der die Cuffblase (Cuff) aus einem flexiblem Weichfolienmaterial geringer Wanddicke besteht. Eine solche Cuffblase ist für vielfältige Anwendungen bei der Intubation und maschinellen Beatmung von Patienten gut geeignet.

Auch die DE 196 38 935 C1 beschreibt eine vergleichbare Trachealbeatmungsvorrichtung, die allgemein angewendet werden kann.

Die US 4502482 beschreibt eine weitere Trachealbeatmungsvorrichtung mit einer Cuffblase

Ein Bereich, in dem die Anwendung von cuff-tragenden Beatmungstuben nach wie vor problematisch ist, ist die tracheale Intubation von Neugeborenen und Kindern. So gelten Kinderbeatmungstuben mit Cuff für den Patienten als ausgesprochen riskant, da durch die Füllung der Cuffblase bedingt immer wieder Schädigungen der Luftröhre (Trachea) und des Kehlkopfes (Larynx) verursacht werden. Die Läsionen werden in der Regel durch die unmittelbare Wirkung des Fülldrucks des Cuffs auf die Durchblutung der versorgenden Gefäße im dem Cuff anliegenden Gewebe hervorgerufen. Die Minderversorgung, Infarzierung bzw. das Absterben der betroffenen Gewebe und Strukturen kann zu äußerst schwerwiegenden lebenslangen Beeinträchtigungen oder zum Versterben des Kindes führen.
Besonders problematisch sind in diesem Zusammenhang Beatmungstuben mit sogenannten low-volume/high-pressure Cuffs. Ihre Cuffblase hat im frei entfalteten, nicht intubierten Zustand einen Durchmesser, der den Durchmesser der zu dichtende Trachea unterschreitet. Zur trachealen Dichtung muß die Wandung des Cuff daher in der Regel unter hohem Druck aufgedehnt werden. Die so auf das anliegende Gewebe wirksam werdenden Dehnungsdrucke führen in aller Regel zu einer völligen Unterbrechung der Gefäßversorgung und bereits nach kürzester Zeit zur Degeneration der dem Cuff anliegenden Strukturen.
Bei den heute verwendeten Trachealtuben werden bei der Gestaltung der Cuffblase bevorzugt dünne Folien verwendet, die in ihrer Dimensionierung residualvolumig ausgebildet sind, sog. high-volume/low-pressure Cuffs. Die Cuffblase hat bei diesen Tuben im frei entfalteten, nicht intubierten Zustand einen Durchmesser, der den Durchmesser der zu intubierenden Trachea deutlich überschreitet (mit einer ausreichenden Sicherheits-Toleranz von in der Regel ca. 50%). Durch die sich beim Befüllen eines solchen im Durchmesser überbemessenen Cuffs in der Luftröhre (Blockung) entwickelnde Einfaltung der Cuffhülle wird bei der trachealen Dichtung mit high-volume/low-pressure Cuffs eine Aufdehnung der Cuffhülle unter potentiell gewebeschädigenden Druckwerten, wie sie bei low-volume/high-pressure Cuffblasen die Regel ist, weitgehend vermieden. Die bewußt hergestellte Einfaltung der Blasenhülle ermöglicht bei der trachealen Dichtung mit highvolume/low-pressure Cuffs demnach perfusions-verträgliche Fülldruckwerte und stellt für den Anwender letztlich sicher, daß der in der Cuffblase gemessene barometrische Druck dem auf das Gewebe transmural übertragenen Druck weitgehend entspricht. Bei der Intubation Erwachsener konnten schwerwiegende tracheale oder laryngeale Schäden durch den Einsatz solcher hochvolumiger Cuffblasen mit in situ eingefalteter Cuff-Hülle auch bei langfristiger Intubation auf ein sehr geringes Maß reduziert werden.

Bei Beatmungstuben für die Intubation von Früh- und Neugeborenen bzw. Kindern und Kleinkindern ist die Anwendung des für Erwachsene bewährten high-volume/low-pressure Prinzips jedoch immer noch problematisch. Mit den heute gebräuchlichen Cuff-Materialien, wie PVC, Latex und Silikon, ist es nicht möglich residualvolumige Cuffblasen zu fertigen, die in Form und Dimension den besonderen Anforderungen der Intubation der kindlichen Luftwege entsprechen und sich vor allem auch bei längerfristiger Intubation zuverlässig atraumatisch verhalten.
So kann eine zur Sicherung des Niedrigdruckverhaltens erforderliche geometrische Ausformung der Ballons bei Verwendung konventioneller Materialien zwar prinzipiell technisch umgesetzt werden, dennoch sind diese Cuffblasen, durch die spezifischen Materialeigenschaften bedingt, für die pädiatrische Beatmung ungeeignet.
So werden entsprechende Cuffblasen aus PVC in der Regel in Wandstärken von 50 bis 100 micron bzw. bei Silikon und Latex in Wandstärken von 100 bis 200 micron gefertigt. Die Verarbeitungsgrenze von PVC zu beatmungstauglichen Cuffblasen (i.d.R. durch online Extrusions-Blowmoulding) liegt bei einer kritischen unteren Wandstärke von etwa 40 bis 50 Micrometern. Werden PVC-Cuffs deutlich dünnwandiger ausgeführt, so geht dies bei bereits geringgradiger Druckbelastung (von bei der trachealen Intubation üblichen 20 bis 30 mbar) mit der Gefahr einer fokalen nichtelastischen Ausstülpung der Cuffwandung (Herniierung) einher, was im ungünstigsten Fall zu einer Verlegung der distalen Öffnung des Beatmungstubus durch die sich ausformende Hernie und zu einem gefürchteten Ventileffekt bei der Beatmung führen kann.
Entsprechendes gilt für die Verarbeitung von Latex zu residualvolumig ausgeformten Ballons unter 100 Mikrometern Wandstärke. Da latex-basierte Cuffblasen im Tauchverfahren hergestellt werden ist die Fertigung dünnwandigerer Ballons unter 100 Mikrometern zum einen technisch schwierig, zum anderen weisen solche Ballons in vielen Fällen unter Beatmungsbedingungen eine Unzureichende mechanische Belastbarkeit auf. Darüber hinaus gelten latex-basierte Komponenten heute aufgrund ihrer potentiellen Allergenität als ungeeignet.
Silikonballons werden ebenfalls im Tauchverfahren hergestellt und sind bei residual ausgeformter Geometrie im Wandstärkenbereich kleiner 100 Mikrometern aus entsprechenden Gründen für Kinderbeatmungstuben nur bedingt verwendbar.
Die genannten bei PVC und Silikon erforderlichen Mindestwandstärken gehen bei der Ausformung eines ausreichend residual bemessnen Cuffs bzw. eines Cuffs geeigneter Geometrie resultieren in aller Regel in einer Mechanik bzw. einer Rigidität der Cuffblase, die sie für eine atraumatische Anwendung bei Kinder-Trachealtuben weitgehend ausschließen. Die für eine atraumatische Kinderintubation erforderlichen besonderen Gestaltungskriterien der Cuffblase wie kleine Radien in der Cuff-Schulter, residualer Durchmesser, zylinderähnliche Ausformung der Cuffblase bei insgesamt kurzer Länge des Zylinders (Cuffs), gehen bei entsprechender Ausführung einer Cuffs in konventionellen Materialien für den pädiatrischen Patienten in mehrfacher Weise mit einem Risiko einher.
So trägt ein entsprechend ausgeformter Cuff aus konventionellem Material mit high-volume/low-pressure tauglicher Bemaßung und zylindrischer Formgebung im evakuierten bzw. entblockten Zustand in aller Regel deutlich prominent, in Faltung liegend auf dem Tubenschaft auf und wird dadurch sowohl bei der Intubation (Einführen des Tubus in die Luftröhre) als auch bei der Extubation (Entfernen des Tubus) zum mechanischen Hindernis. Reflexauslösende Irritationen (Laryngospasmen) des Larynx bzw. der Stimmlippen (Glottis) durch die wülstige, dem Schaft in Faltung aufliegende Cuffhülle können die Folge sein. In vielen Fällen bilden herkömmliche Cuffs im evakuierten Zustand zudem der Schleimhaut zugewandte scharfkantige Umschlagfalten der Cuffhülle aus, die sowohl bei der Intubation als auch bei der Extubation des Cuffs zu schneidenden Verletzungen der Schleimhaut bis hin zur schneidenden Penetration tiefer liegender Strukturen führen können. Im tracheal geblockten Zustand ist bei Kinder-Cuffs konventioneller Bauart, bedingt durch die Materialwandstärke und der aus ihr resultierenden Rigidität, in vielen Fällen zudem keine homogene druckverteilung der Cuffblase auf die Trachealschleimhaut gewährleistet Die Rigidität der Cuffhülle führt bei der Ausbildung der Faltung in situ häufig zur Quetschung und Stauung (Bluterguß) der Schleimhaut im Bereich des der Tracheaiwand zugewandten zwickelförmigen Eintritts der Einfaltung der Cuff-Wandung. Daneben entstehen in vielen Fällen transmural wirksame Druckmaxima innerhalb der Ballonabschnitte, welche zur Trachea hin konvex geformt zwischen den Einstülpungsbereichen der Faltung liegen .und dort fokal zu kritischen, den eigentlichen Fülldruck des Cuffs deutlich überschreitenden Druckwerten auf das anliegende Gewebe mit der Folge entsprechender Minderdurchblutung der anliegenden Schleimhaut (Infarzierung) führen können. Die zur in situ Entfaltung solcher Cuffs herkömmlicher Bauart notwendigen Fülldrucke kommen den kritischen Durchblutungswerten bereits nahe. Das Faltungsmuster einer entsprechend geformten aus konventionellen Materialien gefertigten Cuffblase in der Trachea ist bedingt durch die mangelnde Geschmeidigkeit der Cuff-Hülle in der Regel grob und in seiner Dichtungsleistung gegen von lungenwärts (tracheo-bronchial) anlastendem Gas und rachenwärts anlastendem Sekret nur wenig effizient. Dies ist vor allem dann problematisch, wenn der von tracheo-bronchial am Cuff anlastende Beatmungsdruck den Fülldruck des Cuffs kurzfristig überschreitet (Spitzendrucke). Um ein gewisse Dichtung herzustellen muß der residualvolumig gestaltete Cuff herkömmlicher Bauart in der Regel bereits mit grenzwertig kritischen Drucken befüllt werden bzw. überschreitet diese deutlich.

Kinderbeatmungstuben mit Cuffblase können auf der Basis konventioneller Materialien heute also nur funktionell unzulänglich und potentiell traumatisierend ausgeführt werden. Wegen der schwierigen bzw. nicht möglichen Abstimmung konventioneller Cuff-Materialien mit einer Niedrigdruck-Geometrie bzw. Ausformung des Cuffs, werden die Cuffblasen vieler Kinderbeatmungstuben heute entweder nicht-residual oder nicht ausreichend residual gestaltet (low-volume/high-pressure Cuff). In anderen Fällen weicht der Cuff zur Reduzierung der rigiditätsbedingten, auf dem Schaft im - evakuierten Zustand irritierend bzw. traumatisierend wirkenden Aufwulstung der Cuff-Hülle in seiner Länge von der anatomisch-physiologisch verträglichen Längenausdehnung eines Cuffs deutlich ab. Zur Vermeidung einer solchen Aufwulstung die vor allem im Schulterbereich des Cuffs mit einer besonderen Rigidität einhergeht, wird diesem häufig ausweichend eine angenäherte Spindelform gegeben. Er weist dann zwar in seinem zentralen Abschnitt u.U. einen ausreichend residualen Durchmesser auf, der proximal und distal von der zentralen Portion gelegene spindelförmig ausgezogene Anteil führt jedoch in aller Regel zu einer potentiell traumatisierenden Überlänge des Cuffs. In vielen Fällen reicht die proximale Portion solcher Cuffs in situ in den besonders druckempfindlichen, unterhalb der Stimmlippen (Glottis) gelegenen sogenannten subglottischen Kehlkopf. Bei unsachgemäßer Intubation (zu hohe Plazierung des Cuffs in der Trachea) bzw. bei Verwendung unsachgemäß konstruierter Trachealtuben (überlanger Cuff) entstehen in diesem Abschnitt der Kinderluftwege die gravierendsten und komplikationsträchtigsten Läsionen. Der subglottische Kehlkopf muß daher bei der Gestaltung cuff-tragender pädiatrischer Beatmungstuben Weise als besondere Gefahrenquelle berücksichtigt werden.
Das hohe Gesamt-Anwendungsrisiko herkömmlicher cuff-tragender Kindertuben veranlaßt die überwiegende Mehrzahl der Anwender heute immer noch dazu auf den Cuff als dichtendes Element vollständig zu verzichten. Kinderbeatmungstuben die nicht mit einem dichtenden Cuff versehenen sind werden in diesem Falle in ihrem Außendurchmesser so bemessen, das die Dichtung der Luftwege gegen den positiven Beatmungsdruck im Wesentlichen durch den Schaft des Tubus selbst hergestellt wird. Der Durchmesser des Tubusschaftes wird dabei so gewählt, daß er dem Durchmesser der anatomisch physiologischen Engstelle der unteren Luftwege beim Kind, dem sogenannten Ringknorpel (Krikoid) weitgehend entspricht. Ein kleines Air-Leak wird hierbei in der Regel vom Anwender toleriert bzw. als Sicherheitsfaktor zur Vermeidung gefährlicher Spitzendrucke in der Kinderlunge angestrebt.
Der Kindertrachealtubus ohne dichtende Cuffblase ist jedoch in vielen Fällen bei der Beatmung von Nachteil. Problematisch sind vor allem chirurgische Eingriffe, die eine sehr konstante Narkoseführung (stabiles Beatmungs-Minutenvolumen) bzw. konstante Blutgaswerte verlangen, wie dies zum Beispiel bei der kardiochirurgischen- oder neurochirurgischen intra-operativen Beatmung der Fall sein kann. Im Verlauf einer Intensivbeatmung können spontane Lagerungswechsel des Kindes mit stark schwankenden Air-Leaks einhergehen und bei hoher Vigilanz eine stabile Beatmung unmöglich machen. Bei stark blutenden Eingriffen im Kopfbereich bzw. bei intraoperativer antiseptischer Spülung der Mund- und Rachenhöhle wird in Fällen wegen der unzureichenden Dichtungsleistung eines Tubus ohne Cuff ebenfalls ein cuff-tragender Tubus bevorzugt. Blut, Spülungen und Sekrete des Rachens gelangen ansonsten weitgehend ungehindert in die distalen Luftwege und können den Beatmungsverlauf sowie den Verlauf während und im Anschluß an die Extubation erheblich komplizieren.

### Darstellung der Erfindung

Der Erfindung liegt die Aufgabe zugrunde, einen Trachealtubus mit tracheal dichtender Cuffblase zu schaffen, der zur langfristigen atemwegsverträglichen Verwendung bei Kindern geeignet ist, und das bei den bisher gebräuchlichen, mit einem Cuff ausgestatteten Kinder-Trachealtuben bekannte Traumatisierungsrisiko vermeidet bzw. entscheidend reduziert.

Die Lösung der gestellten Aufgabe wird mit den Merkmalen des Anspruchs 1 erreicht.

Bei einer Trachealbeatmungsvorrichtung der eingangs genannten Art wird der Trachealtubus in erfindungsgemäßer Ausführung für eine jeweilige Alters- bzw. -entwicklungsklasse der Beatmungsphysiologie des Kindes entsprechend mit einem Cuff, der sich durch eine spezifische Kombination von Cuff-Material und Cuff-Wandstärke, sowie Dimensionierung und Positionierung auf dem Tubenschaft auszeichnet, versehen und hergestellt. Der erfindungsgemäße Trachealtubus gewährleistet eine anwendungssichere atraumatische Alternative zum bisher bei der Intubation von Kindern bevorzugten Prinzip der Luftwegsdichtung in Höhe der physiologischen Engstelle der Atemwege (Krikoid) durch einen im Durchmesser angepaßten Tubenschaft. Die Dichtung gegen das Atemgas bzw. sich oberhalb der des Krikoids sammelnde Sekrete wird statt dessen durch eine tracheal plazierte Cuffblase hergestellt. Die Cuffblase kommt beim erfindungsgemäßen Trachealtubus idealerweise im Bereich des Übergangs des distalen zum mittleren Drittel der Luftröhre zu liegen, und stellt dort, durch ihre besonderen Material- und Dimensionierungseigenschaften gewährleistet, eine Dichtung der Luftröhre bei Cuff-Fülldruckwerten (5 bis 15 mbar) her die deutlich unterhalb der Druckwerte der Gewebedurchblutung liegen (30 bis 35 mbar). Der erfindungsgemäße Tubus vermeidet daher mit hoher Wahrscheinlichkeit cuffdruck-bedingte Läsionen der anliegenden Schleimhaut (Quetschungen, Infarzierungen) wie sie bei herkömmlichen Kinderbatmungstuben mit Cuff nicht nur im Bereich der Luftröhre sondern auch im Bereich des in Bezug auf Spätfolgen besonders problematischen subglottischen und glottischen Kehlkopfes bekannt sind.
Bedingt durch die mikrodünnwandige Ausführung der Cuffblase ermöglicht der erfindungsgemäße Tubus eine nahezu wulstfreie Evakuation des Cuffs und vermeidet damit Irritationen oder gar schneidende Verletzungen bei der Intubation und Extubation weitgehend zu vermeiden.
Der erfindungsgemäße Tubus soll zudem in der Lage sein bei Blockung im vorgeschlagenen niedrigen Druckbereich (5 bis 15 mbar) in ausreichendem Umfang gegen Sekret bzw. zuverlässig gegen Gas zu dichten. Er soll unter anderem bei tracheo-bronchial wirksamen Beatmungsdruckwerten (Spitzen- und Plateaudrucke), die den eingestellten Cuff-Fülldruck überschreiten ein zuverlässiges air-seal gewährleisten (Selbstdichtung).
Der erfindungsgemäße Tubus ist in seiner Materialwahl und spezifischen Bemessung so konzipiert, das sich der Anwender bei der Wahl der Tubengröße, die sich bei Beatmungstuben im Allgemeinen am Durchmesser des Schaftes orientiert, von der nach den üblichen Berechnungsformeln errechneten Größe ausgehend, optional für den nächst kleineren, also um 0.5 mm engeren Schaftdurchmesser entscheiden kann. Zur Herstellung der trachealen Dichtung bei Standardbeatmungsbedingungen (Beatmungsdruck < Cuff-Fülldruck) sowie der Selbstdichtung des Cuffs gegen Beatmungsdrucke, die den Fülldruck des Cuff überschreiten, sind .auch bei der optionalen kleineren Schaftgröße die zuvor beschriebenen nichtpesfusionsbeeinträchtigenden Fülldrucke des Cuffs ausreichend. Durch die optionale Wahl eines kleineren Schaftdurchmessers kann der potentiell traumatisierende Effekt eines zu groß gewählten Tubenschaftes (gewebeschädigende Relativbewegungen zwischen Krikoid und Schaft mit gefährlichen Schwellungen des irritierten Gewebes in der Folge) reduziert werden, was dem Anwender zusätzliche Anwendungssicherheit bietet.

Das bevorzugte Folienmaterial der Cuffblase ist ein Polyurethan bzw. ein Polyurethan-Compound. Alternativ kommen Materialien in Frage, die zum einen im erfindungsgemäßen Wandstärkenbereich verarbeitet werden können, zum anderen im angestrebten Fülldruckbereich eine Druck-Volumen-Dehnungsmechanik aufweisen, die der von Polyurethan entspricht.

Die Wandstärke der verwendeten Folie beträgt 0,015 bis 0,005 mm. Es wird eine Wanddicke bevorzugt, die kleiner oder gleich 0,010 mm und größer oder gleich 0.005 mm ist. Als ideal für die erfindungsgemäße atraumatische Dichtung hat sich eine Wandstärke von ca. 0.007 mm erwiesen. Dabei werden die Wandstärken innerhalb der Ballonfolie bevorzugt so ausgebildet, dass sie im dem Tubenschaft angrenzenden Schulter-Bereich dicker ist als in dem der Trachealschleimhaut unmittelbar anliegenden zylindrischen Abschnitt.

Die technische Ausführung der erfindungsgemäßen Cuffs wird im folgenden auf der Basis charakteristischer Relationen bestimmter Kenngrößen, die den Cuff bzw. dessen Plazierung beschreiben, dargelegt. Zur entsprechenden Beschreibung werden verwendet: Durchmesser des frei entfalteten, nicht in der Trachea plazierten Cuffs (D-CUFF), unterer Radius (R1) und oberer Radius (R2) in der Schulterportion des frei entfalteten, nicht auf dem Tubenschaft aufgebrachten Cuffs, Distanz zwischen den beiden Übergangspunkten von R1 zu R2 (L2), Abstand der Montagepunkte des Cuffs auf dem Tubenschaft (MD_MP), Abstand von Tubusspitze und proximalen Cuffmontagepunkt auf dem Schaft (SP_MP), Abstand von Tubusspitze und distalen Cuffmontagepunkt auf dem Schaft (SP_MD), Innendurchmesser des Tubusschaftes (ID), Abstand der Tubusspitze zur glottischen Tiefenmarkierung (SP_GM).
Die beschriebenen Größenrelationen gelten für Kinder-Trachealtuben mit Schaft- Innendurchmessern von 3.0, 3.5, 4.0, 4.5, 5.0, 5.5, 6.0, 6.5 und 7.0 mm. Diese Größenteilung deckt die Alters- und Entwicklungsklassen vom Neugeborenen bis zum jugendlichen Erwachsenen von ca. 15 Jahren ab.
Die Durchmesser der Cuffblase werden in der Weise abgestuft, daß der Durchmesser des Cuffs (D_CUFF) von 8 bis 22 mm reicht.

Zur Gewährleistung einer nicht-perfusionsbeeinträchtigenden langfristig verträglichen trachealen Dichtung der Trachea bei StandardBeatmungsbedingungen ist für den erfindungsgemäßen Trachealtubus neben der geeigneten Materialwahl und Ausführung des Materials in geeigneter Wandstärke, im Wesentlichen die Kombination folgender beiden Relationen entscheidend:
a) Die Relation von Cuffdurchmesser (D_Cuff) zum Abstand der Montagepunkte des Cuffs auf dem Tubusschaft (MD_MP) ein, deren hyperbelförmiger Kurvenverlauf sich über alle Größen hinweg näherungsweise mit der Geraden-Funktion D_CUFF (mm) = 0.75 x MD_MP + 4.00 beschreiben läßt.
b) Die Relation von Tubusspitze zum distalen Montagepunkt (SP_DM) zum Innendurchmesser des Tubusschaftes (ID), welche sich ebenfalls hyperbelförmig darstellt und sich näherungsweise über alle Größen hinweg mit der Geraden-Funktion SP_DM (mm) = 2.36 x ID - 0.86 kennzeichnen läßt. Bei der Bemaßung des erfindungsgemäßen Trachealtubus wird im besonderen berücksichtigt, das die axiale Längenausdehnung des auf dem Schaft montierten Cuffs zum einen so klein wie möglich gewählt wird, um die Distanz zwischen dem proximalen Ende des Cuffs und der Glottis bzw. der glottischen Plazierungsmarke zu maximieren (Reduktion des Risikos der Traumatisierung des druckempfindlichen subglottischen Larynx durch einen passager zur Glottis hin dislozierten Cuff), zum anderen jedoch so groß wie gerade eben erforderlich bemessen wird um in der beschriebenen Kombination von Material, Wandstärke und weiterer Bemaßung des Cuffs, die erfindungsgemäße tracheal verträgliche Dichtung der Luftwege herzustellen.

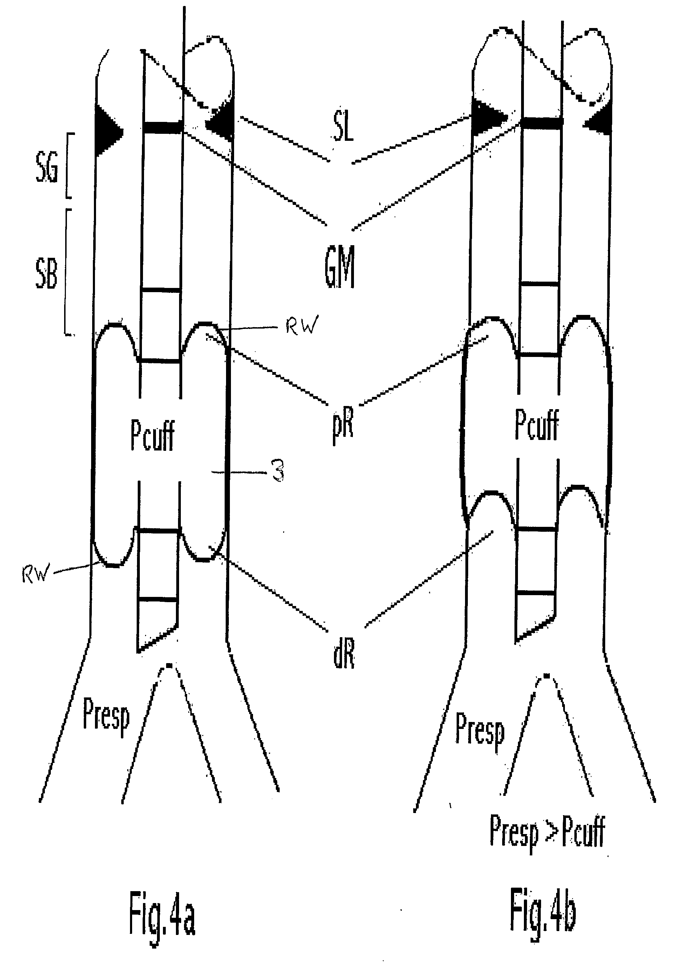

Der Druck in der Cuffblase wird bei erfindungsgemäßer Materialausführung, Bemaßung bzw. Plazierung des Cuffs auf dem Schaft so eingestellt, daß er im Füllbereich von 5 bis 20 mbar und vorzugsweise von 10 bis 15 mbar ein zuverlässiges, schleimhautverträgliches Air-Seal herstellt, welches selbst dann wirksam bleibt wenn der Druck, der sich in den distalen Atemwegen (tracheo-bronchial) unterhalb des Cuffs aufbaut den Fülldruck des Cuffs z.B. in der Plateauphase oder Spitzendruckphase eines Beatmungszyklus kurzfristig überschreitet. Dieses, als sog. Selbstdichtung bezeichnete Verhalten wird durch eine spezifische Ausgestaltung des Cuffs ermöglicht. Der Durchmesser des Cuffs ist soweit residual bemessen (also den Durchmesser der zu dichtenden Trachea überschreitend), um in situ eine nach proximal und distal reichende ringwulstartige Ausformung des gefüllten Cuffs (zwischen Tubusschaft und Trachealwand) zu entstehen zu lassen (siehe Fig. 4a). Überschreitet der Beatmungsdruck den Fülldruck des Cuffs, schlägt der nach distal konvex gerichtete Wulst des Cuffs nach proximal konkav um (siehe Fig. 4b). Wegen des geringen Volumendehnungsverhalten der Cuffhülle bei den zu erwartenden respiratorischen Druckwerten (i.d.R. < 30 mbar) kommt es in dieser Situation, in der sich der am Cuff anlastende Beatmungsdruck auf den Fülldruck des Cuffs überträgt, zu keiner nennenswerten Verformung des proximalen Cuff-Wulstes. Vielmehr übertragen sich die im Cuff vorübergehend entwickelnden Kräfte auf die laterale Wandung (zylindrische Portion) des Cuffs bzw. auf die der lateralen Wandung unmittelbar anliegenden Trachea. Der Cuff schmiegt sich in seiner zylindrischen Portion der Trachealwand mit einer Kraft an, die dem momentan wirksamen Beatmungsdruck entspricht, was bei höherem Beatmungsdruck (20 bis 30 mbar) in aller Regel mit einer erkennbaren Kalibersprung der Trachea im dem Cuff anliegenden Bereich einhergeht.
Zur Ausbildung des Selbstdichtungsverhaltens bei Beatmungssituationen, bei denen der Beatmungsdruck den Fülldruck des Cuffs intermittierend überschreitet, wiest der erfindungsgemäße Trachealtubus die Kombination zweier weiterer charakteristischer Relationen auf, die die für den Selbstdichtungseffekt der Cuffblase entscheidende steile in situ Ausformung seiner distalen und proximalen Schulterportion ermöglichen.
a) Relation von der Distanz zwischen den Montagepunkten des Cuffs (MD_MP) zur Cufflänge der nicht montierten, freien Cuff-Komponente, die sich im Verhältnis MD_MP= L2 - 2 ausdrückt.
b) Relation von D_CUFF zum Radius R1 (R1 beschreibt den Radius des unteren kreisbogenförmigen Übergangs vom Tubenschaft zur Cuff-Schulter) näherungsweise gekennzeichnet durch das Verhältnis R1 (mm) = 0.19 x D_CUFF + 0.39.

Die mikrodünne Ausführung der Cuffhülle verleiht dem befüllten Cuff die nötige Dynamik und mechanischen Eigenschaften um sich in kürzester Zeit unter wechselnden, dem Cuff anlastenden Druckbedingungen selbst dichtend formverändernd an die Trachea zu schmiegen ohne sich soweit elastisch zu verformen (z.B. bei passagerem Beatmungsdruck > Cuffdruck), daß Beatmungsgas in größerem Maße zwischen Trachealwand und Cuff entweichen kann.

Bei einem erfindungsgemäß ausgeführten Cuff treten im tracheal geblockten Zustand zudem weder Quetschungen der Trachealschleimhaut im Einstülpungsbereich der Cuff-Faltung noch durch lokale Druckspitzen im Kontaktbereich von Cuff und Schleimhaut verursachte Infarzierungen auf. Der zwickelförmige Eintrittsbereich der Faltung der residualen Cuffhülle ist bei den mikrodünnen Ballonfolien in seiner Fläche so gering ausgebildet, das er praktisch kein Gewebe aufnehmen bzw. zwischen den eingefalteten Folienanteilen quetschend schädigen kann. Ebenfalls sind in den zwischen den Einstülpungsbereichen gelegenen Cuffblasenabschnitten beim geblockten Cuff keine Inhomogenitäten in der auf der Trachealwand anlastenden Kräfteverteilung festzustellen, es entstehen also keine fokale, möglicherweise Infarzierungen auslösende Druckspitzen.
Schneidende Verletzungen der Schleimhaut beim Ein- und Ausführen des Tubus sind aufgrund der mikrodünnen Wandstärken des Cuffs, der daraus resultierenden Geschmeidigkeit seiner Hülle und des sich nahezu vollständigen Anschmiegens des evakuierten Cuffs ebenfalls nahezu ausgeschlossen.

Die erfindungsgemäße Gestaltung des Cuffs ist übet Trachealtuben hinaus auch auf pädiatrische Tracheostomietuben anwendbar.

### Kurzbeschreibung der Zeichnungen

In den beiliegenden Zeichnungen ist ein Ausführungsbeispiel eines Trachealtubus mit daran angeordneter Cuffblase dargestellt.

Es zeigt:
- Fig. 1:: einen Trachealtubus in der Seitenansicht;
- Fig. 2:: die Form einer frei entfalteten nicht montierten Cuffblase im Schnitt,
- Fig. 3:: auf dem Schaft montierte Cuffblase im Schnitt
- Fig. 4a: Plazierung des Trachealtubus in der Trachea im Schnitt
- Fig. 4b: schematische Darstellung der Selbstdichtungsfunktion
- Fig. 5a-d: graphisch beschreibende Darstellung der erfindungsgemäßen Kenngrößenrelationen

### Ausführung der Erfindung

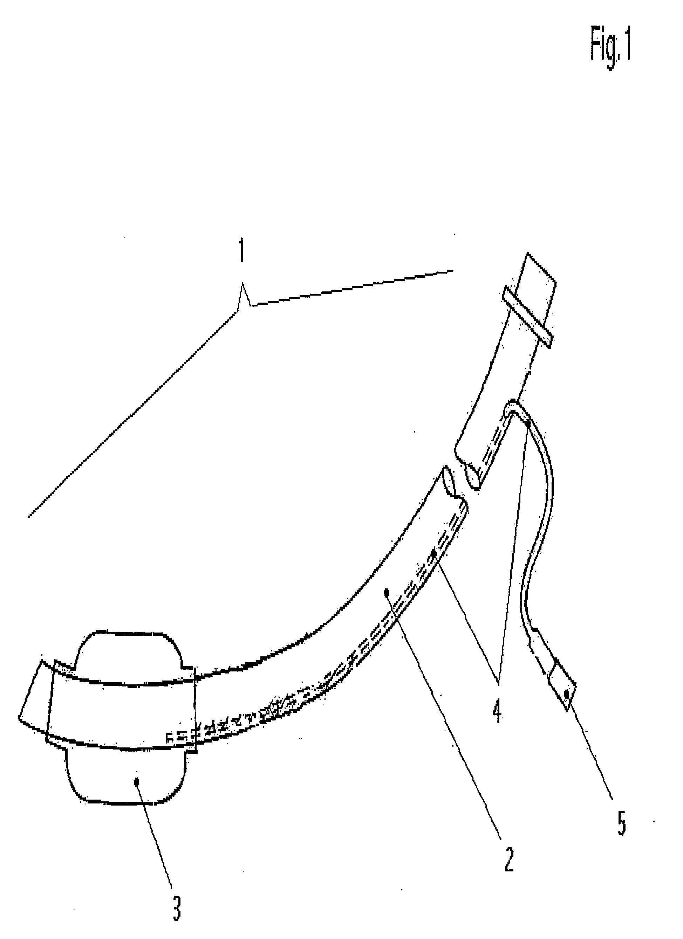

Die Fig. 1 zeigt einen Trachealtubus 1 in der Ansicht. Die Beatmungskanüle 2 ist mit der Cuffblase 3 versehen. Über eine in der Wandung der Kanüle 2 angebrachte Leitung 4 wird die Cuffblase 3 aufgeblasen (geblockt) bzw. die eingeführte Luft daraus abgelassen (entblockt). Hierfür trägt die Leitung 4 an ihrem aus der Kanüle 2 herausgeführten Ende das Ventil 5. Der Trachealtubus 1 ist in Auswahl und Anordnung seiner Komponenten derart ausgebildet, dass er bei allen zu erwartenden Beatmungssituationen eine tracheal gewebeverträgliche Dichtung gewährleistet. Der Trachealtubus 1 wird zur optimalen Erfüllung dieser Aufgabe in mehreren abgestuften Größen ausgeführt.

Die Cuffblase 3 besteht vorzugsweise aus Polyurethan, z.B. aus dem Werkstoff Pellethane 2363 der Firma Dow Chemical Inc.. Es handelt sich hier um ein Polyurethan mit hoher Festigkeit und chemischer Beständigkeit.
Die Wandstärke der Cuffblase beträgt 0,015 bis 0,005 mm. Bevorzugt wird die Wanddicke kleiner oder gleich 0,010 mm ausgeführt. Die Wanddicke der Cufblase beträgt idealerweise ca. 0,007 mm.

Die Volumendehnung der Hülle der Cuffblase vom frei entfalteten, nicht intubierten, nicht mit Druck beaufschlagtem Zustand, wobei der Fülldruck geringgradig über Umgebungsdruck liegt, zu einem Fülldruck von ca. 30 mbar beträgt ca. 5 - 15%, bevorzugt jedoch nicht mehr als 10%.
Die Cuffblase 3 wird in ihrer Gestaltung für die abgestuften Größen individuell geformt und in individuell typischer Weise bzw. Position an der Kanüle 2 befestigt. Materialwahl und Wandstärke der Cuffblase 3 ermöglichen in Kombination mit der jeweiligen geometrischen Ausformung der Cuffblase 3 den erfindungsgemäßen atraumatischen Verschluß der Luftröhre, wobei die Cuffblase 3 sich mit niedrigstem, die Gewebedurchblutung nicht beeinträchtigendem Fülldruck der Trachea organverträglich anschmiegt.
Die Kanüle 2 wird (vorzugsweise aus PVC) mit Innendurchmessern (ID) von 3 bis 7 mm (+/- 0.2mm) gefertigt. Die Größenabstufung der Innendurchmesser erfolgt bevorzugt jeweils in Schritten von 0,5 mm. Die Außendurchmesser der Kanüle 2 sind an die Innendurchmesser ID angepaßt und betragen idealerweise 4,1 bis 9,3 mm (+/-0.2mm).

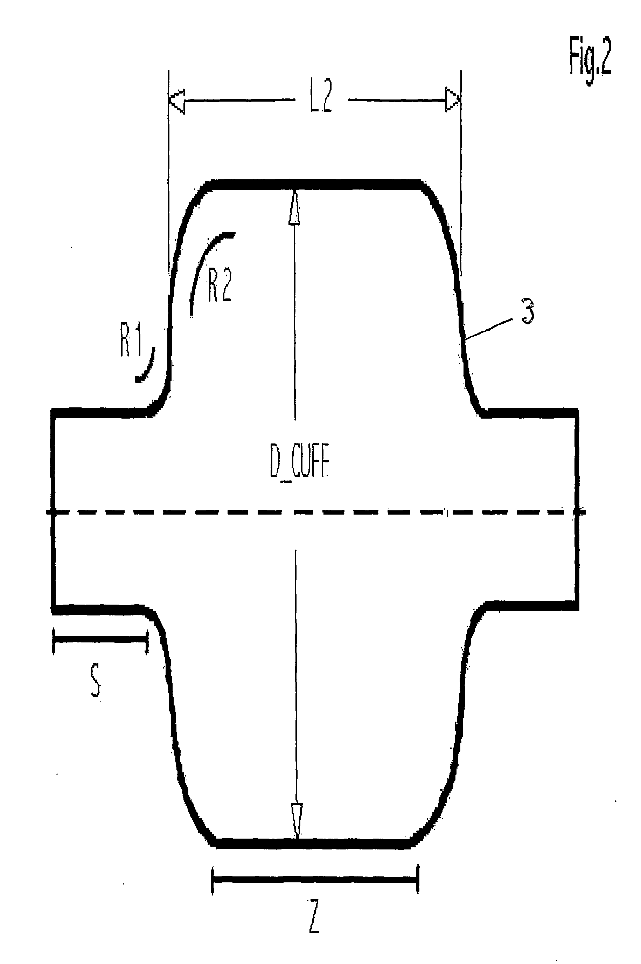

In der Fig. 2 ist die frei entfaltete, noch nicht auf dem Schaft des Tubus montierte Cuffblase als frei stehende Komponente dargestellt. Im leicht aufgeblasen Zustand (geringgradig über Umgebungsdruck) stellen sich über die einzelnen Tubengrößen hinweg folgende Maße dar. Die radiale Ausdehnung der frei entfalteten Cuffblase 3 (D_CUFF) beträgt 10 bis 20 mm. Die axiale Ausdehnung der Cuffblase wird bestimmt durch die Distanz (L2) zwischen den Übergangspunkten von R1 und R2 in der distalen und proximalen Cuff-Schulter. L2 beträgt 10 bis 22 mm. R1 drückt den Radius des kreisbogenförmigen Übergangs von der Schaftportion (S) der Cuffblase in die Cuff-Schulter aus und beträgt 2.55 bis 3.45 mm. R2 bezeichnet den kreisbogenförmigen Übergang der Cuff-Schulter (S) in die der Trachealwand anliegenden zylindrischen Portion (Z). Die Abweichungen der Maße erklären sich jeweils überwiegend durch fertigungsbedingte Schwankungen in der Verarbeitung des Polymers bzw. Elastomers.

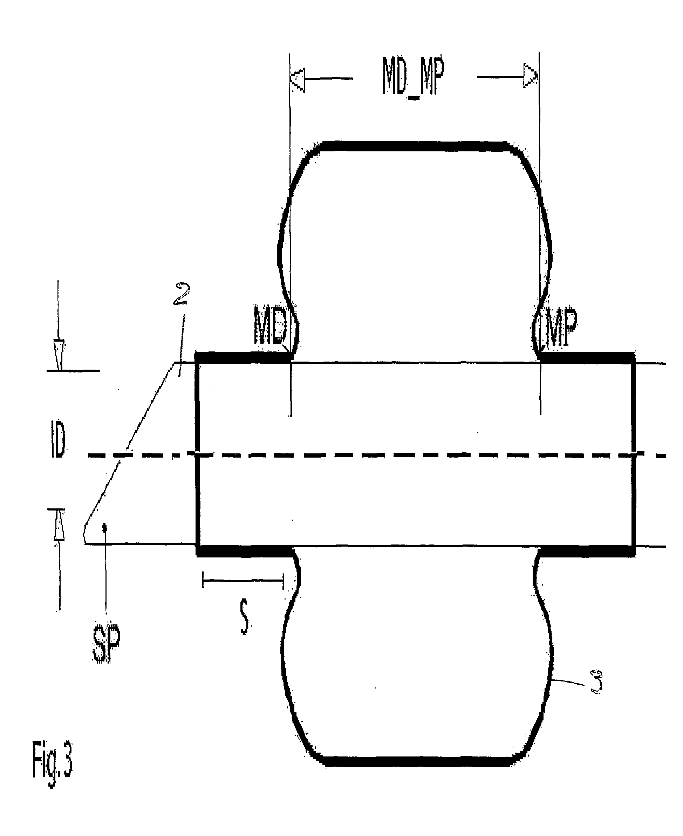

Fig. 3 stellt den auf dem Tubusschaft montierten Cuff in einem schematischen Längsschnitt dar. Die Cuffblase 3 ist auf der Kanüle 2 im Bereich der Schaftportionen (S) der Cuffblase durch vorzugsweise verkleben oder verschweißen fest aufgebracht. MD beschreibt den distalen Montagepunkt der Cuffblase auf der Kanüle. Der Montagepunkt ist definiert durch den Punkt des Übergangs der Schaftportion (S) in den Radius R1 bzw. der Positionierung dieses Punktes auf der Tubuskanüle 2. MP beschreibt entsprechend den proximalen Montagepunkt der Cuffblase.. MD_MP bezeichnet die Distanz zwischen den beiden Montagepunkten auf der Kanüle 2. MD_MP beträgt 8 bis 20 mm (+/-1.5mm). Die Schwankungsbreite der Montagemaße erklärt sich vor allem durch Abweichungen bei der Montage der Cuffblase 3 auf der Kanüle 2. Fig. 4a stellt den Trachealtubus in der Trachea plaziert dar. Die Cuffblase 3 ist im Übergangsbereich vom distalen zum mittleren trachealen Drittel plaziert. Die glottische Markierung (GM) auf dem Tubusschaft (2) beschreibt die korrekte Plazierung des Tubus im Verhältnis zum üblicherweise bei der Intubation verwendeten Orientierungspunkt, den Stimmlippen (SL). SG bezeichnet den sogenannten subglottischen Kehlkopf (Subglottis) der als besonders druck-vulnerabel bekannt ist. Im Bereich des subglottischen Kehlkopfes sind mechanische Irritationen des Gewebes daher so weit wie möglich zu reduzieren. Da bei Lagerungsänderungen oder Spontanbewegungen des Kindes in gewissem Umfang Dislokationen des Tubus bzw. der Cuffblase nach proximal möglich stattfinden, integriert der erfindungsgemäße Trachealtubus einen Sicherheitsbereich (SB) bzw. plaziert den Cuff soweit wie möglich vom subglottischen Kehlkopf entfernt. Die erfindungsgemäßen Dichtungseigenschaften des Tubus bleiben trotz der in ihrer Längenausdehnung minimierten Cuffblase durch deren spezifische Formgebung und Materialkomposition gewährleistet.

Bei der trachealen Blockung des residualvolumigen Cuffs schlägt sich die residual dimensionierte Hülle der Cuffblase in längsverlaufende Falten. Der Cuff bildet zudem in seinen Schulterbereichen nach proximal und distal reichende Ringwülste (RW) aus.

Fig. 4b beschreibt den Selbstdichtungsmechanismus einer erfindungsgemäßen tracheal plazierten Cuffblase bei Beatmungssituationen in denen der Beatmungsdruck den Fülldruck des Cuffs kurzfristig überschreitet. Während der distale Ringwulst (dR) von konvex (Fig.4a) nach konkav (fig.4b) umschlägt, bleibt der proximale Wulst (pR) in seiner Orientierung (konvex) und Form (bedingt durch die geringe Volumendehnung der Cuffhülle) unverändert. Der dem Beatmungsdruck synchron folgende Druckverlauf im Inneren des Cuffs führt statt dessen zu einer moderaten Auswölbung der zylindrischen Portion der Cuffhülle zur Trachealwand hin und sorgt so für eine weitgehend erhaltene Dichtung auch in Spitzendrucksituationen.

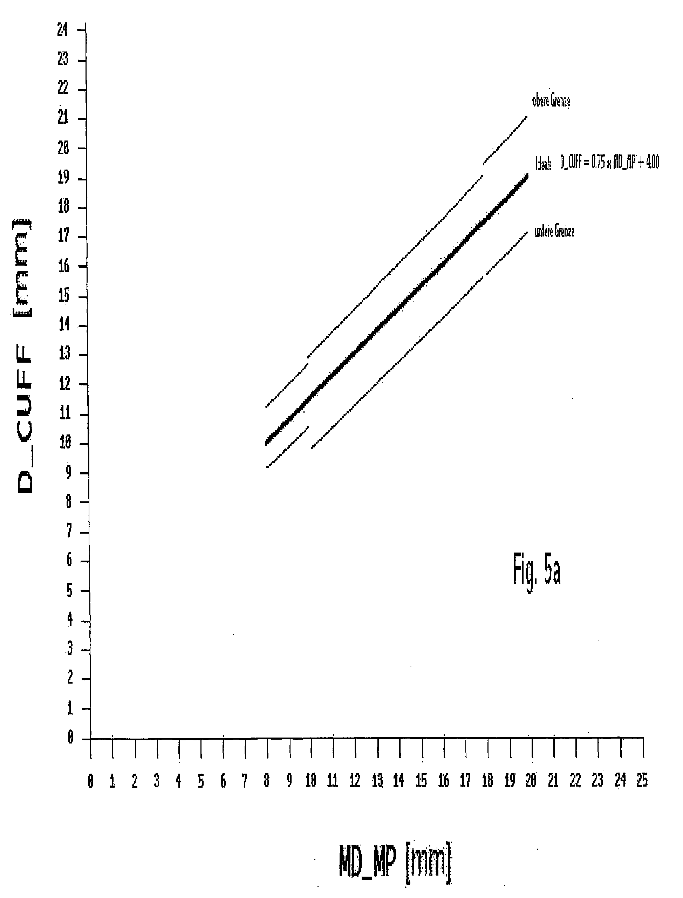

Fig. 5a beschreibt das Verhältnis von D-CUFF zur Distanz zwischen den Montagepunkten MD_MP des Cuffs auf dem Tubenschaft. Die zentrale Gerade (Ideale) gibt den über alle Tuben-Größenbereiche (Innendurchmesser von 3.0 bis 7.0 mm) hinweg geltenden angenäherten Zusammenhang von D_CUFF = 0.75 x MD_MP + 4.00 wieder.
Für Tuben der Größen mit einem Innendurchmesser von 3.0 bis 3.5 ist D_CUFF definiert durch einen Wertebereich, dessen obere Grenze beschrieben ist durch die sich ergebende Gerade aus D_CUFF = 0.75 x MD_MP + 5.00, und die untere Grenze definiert ist durch die Gerade D_CUFF = 0.75 xMD_MP+3.25.
Für Tuben der Größe 4.0 bis 5.5 ergibt sich für D_CUFF ein entsprechender Wertebereich mit der oberen Grenze D_CUFF = 0.75 x MD_MP + 5.20 und der unteren Grenze aus D_CUFF = 0.75 x MD_MP + 2.50.
Bei Tuben der Größen 6.0 bis 7.0 ergibt sich C_CUFF als Wertebereich zwischen der oberen Grenze D_CUFF = 0.75 x MD_MP + 5.50 und der unteren Grenze D_CUFF = 0.75 x MD_MP + 2.50.
Für MD_MP wird dabei, für alle Tubengrößen geltend, eine montagebedingte Toleranz von ca. +/-1.5mm angenommen.

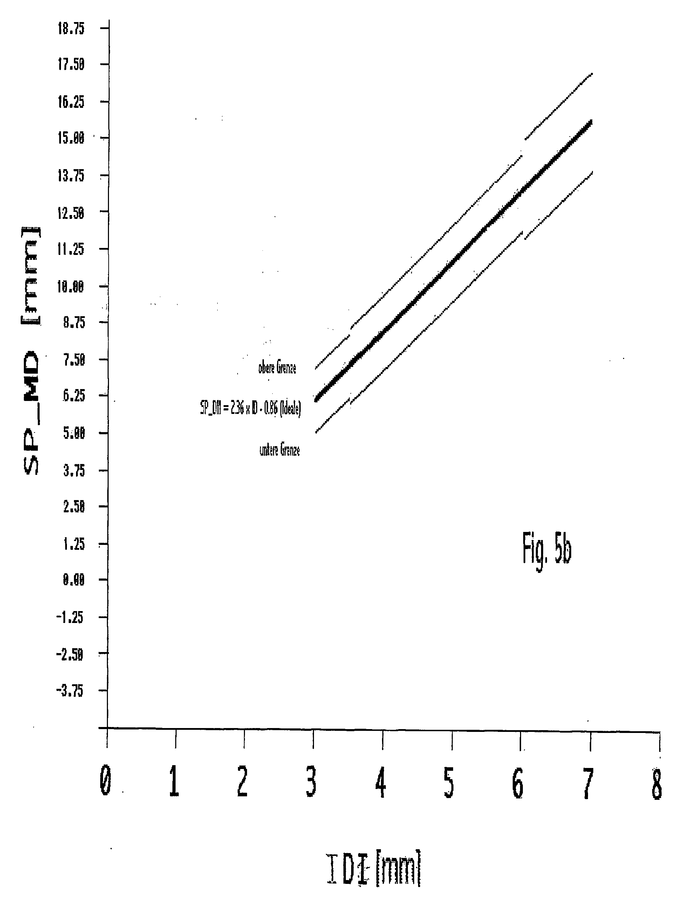

Fig. 5b gibt das Verhältnis von Schaftinnendurchmesser ID und distalem Montagepunkt SP_MD an, welches für alle Tubusgrößen geltend mit der Geraden (Ideale) SP_DM = 2.36 x ID - 0.86 näherungsweise beschrieben werden kann.
Für Tuben der Größen mit einem ID von 3.0 bis 3.5 ist SP_DM in seiner oberen Grenze definiert durch die sich ergebende Gerade aus SP_DM = 2.36 x ID - 0.11, in seiner unteren Grenze durch die Gerade SP_DM = 2.36 x ID - 1.86. Für Tuben der Größe 4.0 bis 5.5 ergibt sich die obere Grenze für SP_DM aus SP_DM = 2.36 x ID +0.34, die untere Grenze aus SP_DM = 2.36 x ID - 2.16. Bei Tuben der Größen 6.0 bis 7.0 ergeben sich die obere Grenze SP_DM = 2.36 x ID + 0.64 und die untere Grenze SP_DM = 2.36 x ID - 2.46.

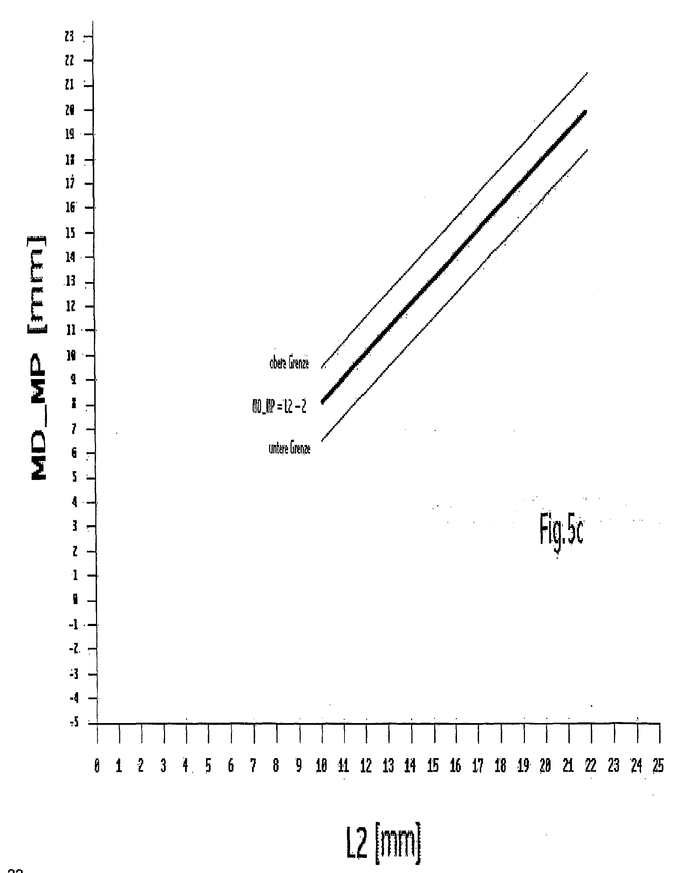

Fig. 5c beschreibt die Relation zwischen der Distanz der Montagepunkte des Cuffs (MD_MP) zur Cufflänge der nicht montierten, frei entfalteten Cuff-Komponente (L2). Das Verhältnis läßt sich für alle Tubengrößen näherungsweise beschreiben mit MD_MP = L2 - 2. Die obere Abweichungsgrenze entspricht über alle Größen hinweg einer Geraden aus MD_MP = L2 - 0.5, die untere aus MD_MP = L2 - 3.5.

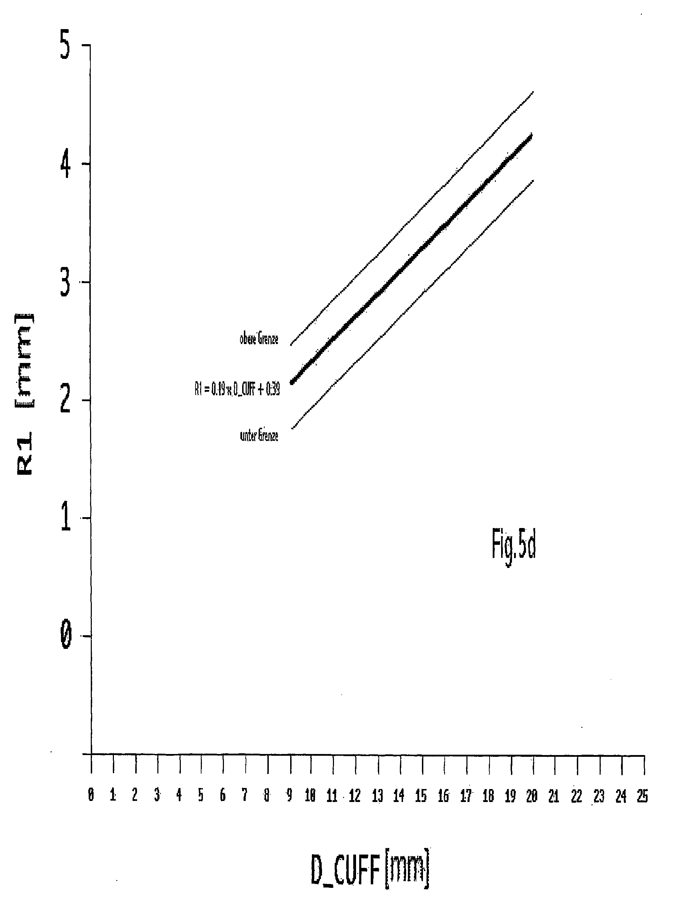

Fig. 5d gibt das Verhältnis von Radius R1 zum Durchmesser D_CUFF für alle Tubengrößen mit der Näherung R1 = 0.19 x D_CUFF + 0.39 wieder. Die obere Abweichungsgrenze entspricht über alle Größen hinweg einer Geraden aus R1 = 0.19 x D_CUFF + 0.69, die untere aus R1 = 0.19 x D_CUFF + 0.09.

## Patentansprüche

1. Trachealbeatmungsvorrichtung (1), welche die Trachea zum Beatmen eines pädiatrischen Patienten möglichst dicht verschließt, mit einer die Trachea unterhalb der Glottis blockierenden Cuffblase (3), durch die eine Beatmungskanüle (2) hindurchgeführt ist, wobei die Cuffblase (3) aus einem flexiblen Weichfolienmaterial besteht und in gefülltem ohne Begrenzung frei entfaltetem Zustand größer ist als in gefülltem Zustand in der Trachea platziert und die Cuffblase (3) mit Faltenwurf an der Trachea anliegt, die Trachealtube (2) einen Schaft-Innendurchmesser von 3,0 bis 7,0 mm aufweist, und der Abstand der Montagepunkte des Cuffs auf dem Tubusschaft (MD_MP) 8-20 mm ± 1.5 mm beträgt, **dadurch gekennzeichnet, dass**
(a) für Trachealtuben (2) mit Schaft-Innendurchmessem (ID) von 3,0 bis 3,5 mm der Durchmesser des freien Cuffs (D_CUFF) einen Wertebereich darstellt, welcher zwischen den Geraden D_CUFF = 0,75 x MD_MP + 5,00 und D_CUFF = 0,75 x MD_MP + 3,50 liegt, sowie der Abstand von Tubusspitze und distalem Cuffmontagepunkt auf dem Schaft (SP_MD) einen Wertebereich darstellt, der zwischen den Geraden SP_MD = 2,36 x ID - 0,11 und SP_MD = 2,36 x ID - 1,86 liegt;
(b) für Trachealtuben (2) mit Schaft-Innendurchmessem (ID) von 4,0 bis 5,5 mm der D_CUFF einen Wertebereich darstellt, welcher zwischen den Geraden D_CUFF = 0,75 x MD_MP + 5,20 und D_CUFF = 0,75 x MD_MP + 2,50 liegt, sowie der SP_MD einen Wertebereich darstellt, der zwischen den Geraden SP_MD = 2,36 x ID - 0,34 und SP_MD = 2,36 x ID - 2,16 liegt; und
(c) für Trachealtuben (2) mit Schaft-Innendurchmessem (ID) von 6,0 bis 7,0 mm der D_CUFF einen Wertebereich darstellt, welcher zwischen den Geraden D_CUFF = 0,75 x MD_MP + 5,50 und D_CUFF = 0,75 x MD_MP + 2,50 liegt, sowie der SP_MD einen Wertebereich darstellt, der zwischen den Geraden SP_MD = 2,36 x ID - 0,64 und SP_MD = 2,36 x ID - 2,46 liegt.

2. Trachealbeatmungsvorrichtung (1) nach Anspruch 1, **dadurch gekennzeichnet, dass** sich für Trachealtuben (2) mit Schaft-Innendurchmessern (ID) von 3.0 bis 7.0 mm für D_CUFF ein Verhältnis D_CUFF = 0.75 x MD_MP + 4.0, sowie für den SP_MD ein Verhältnis SP_MD = 2.36 x ID - 0.86 einstellt.

3. Trachealbeatmungsvorrichtung (1) nach Anspruch 1 oder 2, **dadurch gekennzeichnet, dass** sich für Trachealtuben (2) mit Schaft-Innendurchmessern (ID) von 3.0 bis 7.0 mm für den MD_MP näherungsweise ein Verhältnis von MD_MP = L2 - 2 darstellt, wobei L2 die Distanz zwischen den beiden Übergangspunkten der Radien R1 und R2 in der Schulterportion des frei entfalteten Cuffs ist.

4. Trachealbeatmungsvorrichtung (1) nach Anspruch 1 oder 2, **dadurch gekennzeichnet, dass** MD_MP in einem Wertebereich zwischen den Geraden MD_MP = L2 - 0.5 sowie MD_MP = L2 - 3.5 liegt.

5. Trachealbeatmungsvorrichtung (1) nach Anspruch 1 oder 2, **dadurch gekennzeichnet, dass** sich für Trachealtuben (2) mit Schaft-Innendurchmessernm von 3.0 bis 7.0 mm für R1 ein Verhältnis von R1 = 0.19 x D_CUFF + 0.39 einstellt.

6. Trachealbeatmungsvorrichtung (1) nach Anspruch 1 oder 2, **dadurch gekennzeichnet, dass** sich für Trachealtuben (2) mit Schaft-Innendurchmessern von 3.0 bis 7.0 mm für R1 ein Wertebereich ergibt der zwischen den Geraden aus R1 = 0.19 x D_CUFF + 0.69 und R1 = 0.19 x D_CUFF + 0.09 liegt.

7. Trachealbeatmungsvorrichtung (1) nach Anspruch 1 oder 2, **dadurch gekennzeichnet, dass** sich für Trachealtuben (2) mit Schaft-Innendurchmessern von 3.0 bis 7.0 mm für MD_MP ein Verhältnis von MD_MP = L2 - 2, sowie für R1 ein Verhältnis von R1 = 0.19 x D_CUFF + 0.39 einstellt.

8. Trachealbeatmungsvorrichtung (1) nach Anspruch 1 oder 2, **dadurch gekennzeichnet, dass** sich für Trachealtuben (2) mit Schaft-Innendurchmessern von 3.0 bis 7.0 mm für MD_MP ein Wertebereich zwischen den Geraden MD_MP = L2 - 0.5 sowie MD_LP = L2 - 3.5, sowie für R1 ein Wertebereich zwischen den Geraden R1 = 0.19 x D_CUFF + 0.69 und R1 = 0.19 x D_CUFF + 0.09 ergibt.

9. Trachealbeatmungsvorrichtung (1) nach einem der Ansprüche 1 bis 8, **dadurch gekennzeichnet, dass** die Wanddicke (D) der Folie (7) 0,015 bis 0,005 mm beträgt.

10. Trachealbeatmungsvorrichtung (1) nach einem der Ansprüche 1 bis 9, **dadurch gekennzeichnet, dass** die Wanddicke (D) der Folie (7) kleiner oder gleich 0,01 mm ist.

11. Trachealbeatmungsvorrichtung (1) nach einem der Ansprüche 1 bis 10, **dadurch gekennzeichnet, dass** die Wanddicke (D) der Folie (7) im Bereich des Faltenwurfs dünner ist als in dem zur Kanüle (2) gerichteten faltenfreien Bereich.

12. Trachealbeatmungsvorrichtung (1) nach einem der Ansprüche 1 bis 11, **dadurch gekennzeichnet, dass** die Folie (7) der Cuffblase (3) aus einem Polyurethan besteht.

13. Trachealbeatmungsvorrichtung (1) nach einem der Ansprüche 1 bis 12, **dadurch gekennzeichnet, dass** die Kanüle (2) mit abgestuften Innendurchmessern (ID) von 3 bis 7 mm vorliegt,

14. Trachealbeatmungsvorrichtung (1) nach Anspruch 13, **dadurch gekennzeichnet, dass** die Abstufung der Innendurchmesser (ID) 0,5 mm beträgt.

15. Trachealbeatmungsvorrichtung (1) nach einem der Ansprüche 1 bis 14, **dadurch gekennzeichnet, dass** die Außendurchmesser (OD) der Kanüle (2) an dessen Innendurchmesser (ID) angepasst von 4,1 bis 9,3 mm betragen.

16. Trachealbeatmungsvorrichtung (1) nach einem der Ansprüche 1 bis 15, **dadurch gekennzeichnet, dass** die Cuffblase (3) abgestufte Außendurchmesser (M) von 10 bis 20 mm hat.

17. Trachealbeatmungsvorrichtung (1) nach Anspruch 16, **dadurch gekennzeichnet, dass** die Abstufung des Außendurchmessers (M) der Cuffblase (3) in jeweils zwei gleichbleibenden Stufen erfolgt.

18. Trachealbeatmungsvorrichtung (1) nach einem der Ansprüche 1 bis 17, **dadurch gekennzeichnet, dass** die axiale Länge (N) der Cuffblase (3) 16 bis 32 mm beträgt.

19. Trachealbeatmungsvorrichtung (1) nach einem der Ansprüche 1 bis 18, **dadurch gekennzeichnet, dass** die am Kehlkopf anliegende wirksame Außenfläche der Cuffblase (3) eine axiale Länge (L2) von 10 bis 22 mm hat.

20. Trachealbeatmungsvorrichtung (1) nach einem der Ansprüche 1 bis 19, **dadurch gekennzeichnet, dass** die Abstufung der axialen Länge (L2) in jeweils zwei gleichbleibenden Stufen erfolgt.

21. Trachealbeatmungsvorrichtung (1) nach einem der Ansprüche 1 bis 20, **dadurch gekennzeichnet, dass** das über die Cuffblase (3) hinaus stehende Ende (6) der Beatmungskanüle (2) 4 bis 11 mm beträgt.

22. Trachealbeatmungsvorrichtung (1) nach einem der Ansprüche 1 bis 21, **dadurch gekennzeichnet, dass** an der Kanüle (2) eine Markierung (8) angebracht ist, die den Abstand des oberen Rands der Cuffblase (3) zur Stimmlippe angibt.

23. Trachealbeatmungsvorrichtung (1) nach einem der Ansprüche 1 bis 22, **dadurch gekennzeichnet, dass** der Druck in der Cuffblase (3) im Bereich von 5 bis 20 mbar, vorzugsweise im Bereich von 10 bis 15 mbar, liegt.

## Claims

1. A tracheal ventilation device (1) which seals a trachea in order to ventilate a pediatric patient, the device comprising a cuff balloon (3) that blocks the trachea below the glottis and is traversed by a ventilation cannula (2), said cuff balloon (3) being made of a flexible soft film material, said cuff balloon (3) in a fully inflated state in which said cuff balloon (3) is freely deployed without restriction being larger than when said cuff balloon (3) is in a filled state placed in the trachea, and said cuff balloon (3) lying with folds against the trachea, the tracheal tube (2) having a shaft inner diameter of 3.0 to 7.0 mm, and the spacing of the mounting points of said cuff balloon on the tube shaft (MD_MP) being 8-20 mm ± 1.5 mm, **characterized in that**
(a) for tracheal tubes (2) with shaft inner diameters (ID) between 3.0 and 3.5 mm the fully inflated diameter (D_CUFF) of said cuff balloon has a range of values that lies between straight lines D_CUFF=0.75xMD_MP+5.00 and D_CUFF=0.75xMD_MP+3.50 and the distance between the tip of the tube and a distal mounting point on the tube shaft (SP_MD) has a range of values that lies between straight lines SP_MD=2.36xID-0.11 and SP_MD=2.36xID-1.86;
(b) for tracheal tubes (2) with shaft inner diameters (ID) between 4.0 to 5.5 mm D_CUFF has a range of values between the straight lines D_CUFF=0.75xMD_MP+5.20 and D_CUFF=0.75xMD_MP+2.50 and SP_MD has a range of values between the straight lines SP_MD=2.36xID-0.34 and SP_MD=2.36xID-2.16; and
(c) wherein for tracheal tubes (2) with an inner diameter (ID) of 6.0 to 7.0 mm D_CUFF has a range of values that lies between the straight lines D_CUFF=0.75xMD_MP+5.50 and D_CUFF=0.75xMD_MP+2.50 and SP_MD has a range of values between the straight lines SP_MD=2.36xID-0.64 and SP_MD=2.36xID-2.46.

2. The tracheal ventilation device (1) as specified in claim 1, **characterized in that** for tracheal tubes (2) with an inner diameter (ID) of 3.0 to 7.0 mm, D_CUFF is defined by D_CUFF=0.75xMD_MP+4.0, and SP_MD is defined by a relation of SP_MD=2.36xID-0.86.

3. The tracheal ventilation device (1) in accordance with claim 1 or 2, **characterized in that** for tracheal tubes (2) with shaft inner diameters (ID) of 3.0 to 7.0 mm, MD_MP is approximately defined by a relation MD_MP=L2-2, L2 being the distance between the two transition points of radii R1 and R2 in the shoulder portion of the freely deployed cuff (3).

4. The tracheal ventilation device (1) in accordance with claim 1 or 2, **characterized in that** MD_MP falls within a range of values between the straight lines MD_MP=L2-0.5 and MD_MP=L2-3.5.

5. The tracheal ventilation device (1) in accordance with claim 1 or 2, **characterized in that** for tracheal tubes (2) with shaft inner diameters of 3.0 to 7.0 mm, a relation for R1 is defined by R1=0.19xD_CUFF+0.39.

6. The tracheal ventilation device (1) in accordance with claim 1 or 2, **characterized in that** for tracheal tubes (2) with shaft inner diameters of 3.0 to 7.0 mm, a range of values is obtained for R1 that lies between the straight lines defined by R1=0.19xD_CUFF+0.69 and R1=0.19xD_CUFF+0.09.

7. The tracheal ventilation device (1) in accordance with claim 1 or 2, **characterized in that** for tracheal tubes (2) with shaft inner diameters of 3.0 to 7.0 mm, MD_MP is defined by the relation MD_MP=L2-2, and R1 by the relation R1=0.19xD_CUFF+0.39.

8. The tracheal ventilation device (1) in accordance with claim 1 or 2, **characterized in that** for tracheal tubes (2) with shaft inner diameters of 3.0 to 7.0 mm, MD_MP has a range of values that is between the straight lines MD_MP=L2-0.5 and MD_MP=L2-3.5, and R1 a range of values between the straight lines R1=0.19xD_CUFF+0.69 and R1=0.19xD8CUFF+0.09.

9. The tracheal ventilation device (1) in accordance with any of claims 1 to 8, **characterized in that** a wall thickness (D) of the film (7) is 0.015 to 0.005 mm.

10. The tracheal ventilation device (1) in accordance with any of claims 1 to 9, **characterized in that** a wall thickness (D) of the film (7) is no more than 0.01 mm.

11. The tracheal ventilation device (1) in accordance with any of claims 1 to 10, **characterized in that** the wall thickness (D) of the film (7) in a region of the folds is thinner than in a fold-free region facing the cannula (2).

12. The tracheal ventilation device (1) in accordance with any of claims 1 to 11, **characterized in that** the film (7) of said cuff balloon (3) is made of polyurethane.

13. The tracheal ventilation device (1) in accordance with any of claims 1 to 12, **characterized in that** said cannula (2) is present with graduated inner diameters (ID) of 3 to 7 mm.

14. The tracheal ventilation device (1) as specified in claim 13, **characterized in that** the graduation of the inner diameter (ID) is 0.5 mm.

15. The tracheal ventilation device (1) in accordance with any of claims 1 to 14, **characterized in that** outer diameters (OD) of said cannula (2), adapted to the inner diameters (ID) thereof, are from 4.1 to 9.3 mm.

16. The tracheal ventilation device (1) in accordance with any of claims 1 to 15, **characterized in that** said cuff balloon (3) possesses graduated outer diameters (M) of 10 to 20 mm.

17. The tracheal ventilation device (1) in accordance with claim 16, **characterized in that** the graduation of the outer diameter (M) of said cuff balloon (3) is effected in two equal steps in each case.

18. The tracheal ventilation device (1) in accordance with any of claims 1 to 17, **characterized in that** an axial length (N) of said cuff balloon (3) is 16 to 32 mm.

19. The tracheal ventilation device (1) in accordance with any of claims 1 to 18, **characterized in that** an operative outer face of said cuff balloon (3) lying against the larynx has an axial length (L2) of 10 to 22 mm.

20. The tracheal ventilation device (1) in accordance with any of claims 1 to 19, **characterized in that** the graduation of the axial length (L2) is effected in two equal steps in each case.

21. The tracheal ventilation device (1) in accordance with any of claims 1 to 20, **characterized in that** an end (6) of said ventilation cannula (2) that projects beyond said cuff balloon (3) measures 4 to 11 mm.

22. The tracheal ventilation device (1) in accordance with any of claims 1 to 21, **characterized in that** applied to said cannula (2) is a marking (8) indicating the distance from an upper edge of said cuff balloon (3) to a vocal fold.

23. The tracheal ventilation device (1) in accordance with any of claims 1 to 22, **characterized in that** the pressure in said cuff balloon (3) is in the range of 5 to 20 mbar, preferably in the range of 10 to 15 mbar.

## Revendications

1. Dispositif de respiration trachéale (1) qui ferme de façon aussi étanche que possible la trachée pour la respiration d'un patient pédiatrique, comprenant un ballonnet (3) bloquant la trachée sous la glotte, au travers duquel est guidée une canule respiratoire (2), dans lequel le ballonnet (3) est constitué d'un matériau en feuille souple flexible qui, à l'état rempli librement déployé sans limite, est plus gros qu'à l'état rempli, placé dans la trachée, et le ballonnet (3) est appliqué par plissage sur la trachée, le tube trachéal (2) présentant un diamètre interne de tige de 3,0 à 7,0 mm, et l'espacement des points de montage du ballonnet sur la tige tubulaire (MD_MP) est de 8 à 20 mm ± 1,5 mm, **caractérisé en ce que**
(a) pour des tubes trachéaux (2) présentant des diamètres internes de tige (ID) de 3,0 à 3,5 mm, le diamètre du ballonnet libre (D_CUFF) représente une plage de valeurs qui se situe entre les droites D_CUFF = 0,75 x MD_MP + 5,00 et D_CUFF = 0,75 x MD_MP + 3,50 et l'espacement de la pointe tubulaire et du point de montage de ballonnet distal sur la tige (SP_MD) représente une plage de valeurs qui se situe entre les droites SP_MD = 2,36 x ID - 0,11 et SP_MD = 2,36 x ID - 1,86 ;
(b) pour des tubes trachéaux (2) présentant des diamètres internes de tige (ID) de 4,0 à 5,5 mm, D_CUFF représente une plage de valeurs qui se situe entre les droites D_CUFF = 0,75 x MD_MP + 5,20 et D_CUFF = 0,75 x MD_MP + 2,50 et SP_MD représente une plage de valeurs qui se situe entre les droites SP_MD = 2,36 x ID - 0,34 et SP_MD = 2,36 x ID - 2,16 ; et
(c) pour des tubes trachéaux (2) présentant des diamètres internes de tige (ID) de 6,0 à 7,0 mm, D_CUFF représente une plage de valeurs qui se situe entre les droites D_CUFF = 0,75 x MD_MP + 5,50 et D=CUFF = 0,75 x MD_MP + 2,50 et SP_MD représente une plage de valeurs qui se situe entre les droites SP_MD = 2,36 x ID - 0,64 et SP_MD = 2,36 x ID - 2,46.

2. Dispositif de respiration trachéale (1) selon la revendication 1, **caractérisé en ce que** pour des tubes trachéaux (2) présentant des diamètres internes de tige (ID) de 3,0 à 7,0 mm, on a pour D_CUFF un rapport de D_CUFF = 0,75 x MD_MP + 4,0 et pour SP_MD, un rapport SP_MD = 2,36 x ID - 0,86.

3. Dispositif de respiration trachéale (1) selon la revendication 1 ou 2, **caractérisé en ce que** pour des tubes trachéaux (2) présentant des diamètres internes de tige (ID) de 3,0 à 7,0 mm, pour MD_MP, on a approximativement un rapport de MD_MP = L2 - 2, où L2 est la distance entre les deux points de transition des rayons R1 et R2 dans la partie d'épaulement du ballonnet déployé librement.

4. Dispositif de respiration trachéale (1) selon la revendication 1 ou 2, **caractérisé en ce que** MD_MP se situe dans une plage de valeurs entre les droites MD_MP = L2 - 0,5 et MD_MP = L2 - 3,5.

5. Dispositif de respiration trachéale (1) selon la revendication 1 ou 2, **caractérisé en ce que** pour des tubes trachéaux (2) présentant des diamètres internes de tige (ID) de 3,0 à 7,0 mm, on a pour R1 un rapport de R1 = 0,19 x D_CUFF + 0,39.

6. Dispositif de respiration trachéale (1) selon la revendication 1 ou 2, **caractérisé en ce que** pour les tubes trachéaux (2) présentant des diamètres internes de tige (ID) de 3,0 à 7,0 mm, on a pour R1 une plage de valeurs qui se situe entre les droites de R1 = 0,19 x D_CUFF + 0,69 et R1 = 0,19 x D_CUFF + 0,09.

7. Dispositif de respiration trachéale (1) selon la revendication 1 ou 2, **caractérisé en ce que** pour des tubes trachéaux (2) présentant des diamètres internes de tige de 3,0 à 7,0 mm, on a pour MD_MP, un rapport de MD_MP = L2 - 2 et pour R1, un rapport de R1 = 0,19 x D_CUFF + 0,39.

8. Dispositif de respiration trachéale (1) selon la revendication 1 ou 2, **caractérisé en ce que** pour des tubes trachéaux (2) présentant des diamètres internes de tige de 3,0 à 7,0 mm, on a pour MD_MP, une plage de valeurs entre les droites MD_MP = L2 - 0,5 et MD_LP = L2 - 3,5 et pour R1, une plage de valeurs entre les droites R1 = 0,19 x D_CUFF + 0,69 et R1 = 0,19 x D_CUFF + 0,09.

9. Dispositif de respiration trachéale (1) selon l'une des revendications 1 à 8, **caractérisé en ce que** l'épaisseur de paroi (D) de la feuille (7) est de 0,015 à 0,005 mm.

10. Dispositif de respiration trachéale (1) selon l'une des revendications 1 à 9, **caractérisé en ce que** l'épaisseur de paroi (D) de la feuille (7) est inférieure ou égale à 0,01 mm.

11. Dispositif de respiration trachéale (1) selon l'une des revendications 1 à 10, **caractérisé en ce que** l'épaisseur de paroi (D) de la feuille (7) dans la zone du plissage est inférieure à celle de la zone non plissée tournée vers la canule (2).

12. Dispositif de respiration trachéale (1) selon l'une des revendications 1 à 11, **caractérisé en ce que** la feuille (7) du ballonnet (3) est constituée d'un polyuréthanne.

13. Dispositif de respiration trachéale (1) selon l'une des revendications 1 à 12, **caractérisé en ce que** la canule se présente avec des diamètres internes (ID) étagés de 3 à 7 mm.

14. Dispositif de respiration trachéale (1) selon la revendication 13, **caractérisé en ce que** l'étagement des diamètres internes (ID) est de 0,5 mm.

15. Dispositif de respiration trachéale (1) selon l'une des revendications 1 à 14, **caractérisé en ce que** le diamètre externe (OD) de la canule (2) est adapté à son diamètre interne (ID) de 4,1 à 9,3 mm.

16. Dispositif de respiration trachéale (1) selon l'une des revendications 1 à 15, **caractérisé en ce que** le diamètre externe (M) étagé du ballonnet (3) a 10 à 20 mm.

17. Dispositif de respiration trachéale (1) selon la revendication 16, **caractérisé en ce que** l'étagement du diamètre externe (M) du ballonnet (3) s'effectue respectivement en deux paliers invariables.

18. Dispositif de respiration trachéale (1) selon l'une des revendications 1 à 17, **caractérisé en ce que** la longueur axiale (N) du ballonnet (3) est de 16 à 32 mm.

19. Dispositif de respiration trachéale (1) selon l'une des revendications 1 à 18, **caractérisé en ce que** la surface active du ballonnet (3) qui appuie sur le larynx a une longueur axiale (L2) de 10 à 22 mm.

20. Dispositif de respiration trachéale (1) selon l'une des revendications 1 à 19, **caractérisé en ce que** l'étagement de la longueur axiale (L2) s'effectue respectivement en deux paliers invariables.

21. Dispositif de respiration trachéale (1) selon l'une des revendications 1 à 20, **caractérisé en ce que** l'extrémité (6) de la canule respiratoire (2) sortant au dessus du ballonnet (3) est de 4 à 11 mm.

22. Dispositif de respiration trachéale (1) selon l'une des revendications 1 à 21, **caractérisé en ce qu'**une marque (8) est apposée sur la canule (2), qui indique la distance du bord supérieur du ballonnet (3) aux cordes vocales.

23. Dispositif de respiration trachéale (1) selon l'une des revendications 1 à 22, **caractérisé en ce que** la pression dans le ballonnet (3) est de l'ordre de 5 à 20 mbar, de préférence, de l'ordre de 10 à 15 mbar.
